# EUROPEAN PATENT APPLICATION

(11) **EP 4 806 480 A1**
(43) Date of publication of application: **16.09.2026**
(21) Application number: 25305346.6
(22) Date of filing: 14.03.2025
(51) Int. Cl.: A61M 5/315

(54) **STOPPER FOR USE IN AN INJECTION DEVICE**

(71) Applicant: Becton Dickinson France, 38800 Le Pont-de-Claix (FR)
(72) Inventor: LAVIGNE, Ferdinand, 38170 Seyssinet-Pariset (FR); PILOY, Jefferson, 38000 GRENOBLE (FR); BESSON, Nicolas, 38650 TREFFORT (FR)
(74) Representative: Germain Maureau

(57) **Abstract**

Provided herein is a stopper for use in an injection device, with the stopper including one or more features thereon that address issues related to container closure integrity, gliding force, stopper deformation, minimization of dead volume in the injection device, and stickiness of the stopper. The stopper may include vertical ribs, a patterned roof portion, a proximal shoulder portion with projections thereon, and/or one or more annular lips, with a combination of one or more of these features addressing the aforementioned issues.

## Description

### BACKGROUND OF THE INVENTION

### Field of the Invention

The present disclosure relates generally to a stopper for use with an injection device and, more particularly, to a stopper with features thereon that address issues related to container closure integrity, gliding force, stopper deformation, minimization of dead volume in the injection device, and stopper stickiness.

### Description of Related Art

Medical injection devices, such as syringes and auto-injectors, are used in a variety of environments for administering liquids (e.g., medications or drugs) to a patient. With regard to syringes, many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper or plunger stopper is typically made of elastomeric material and is adapted to ensure the container closure integrity of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The head portion of the stopper may include a plurality of annular, outwardly protruding ribs formed on an external cylindrical wall thereof, with the ribs forming a seal with the syringe barrel to ensure the container closure integrity of the syringe. A series of two or three ribs, for example, may be spaced apart longitudinally along the stopper main body, with each pair of adjacent ribs separated by an inter-rib region provided therebetween.

When designing/providing stoppers for use in syringes and/or auto-injectors, it is recognized that many design and use factors must be considered. As one example, it is required for a stopper to prevent leaks and maintain closure integrity in the syringe, while providing an acceptable gliding force for moving the stopper. As another example, it is desirable for a stopper to have limited deformation (e.g., radial deformation) during use and during insertion thereof (e.g., during a vent tube stoppering process), so as to maintain desirable gliding characteristics when performing an injection and/or to prevent tilting of the stopper during insertion. As yet another example, it is desirable for a stopper to be configured to limit/minimize dead volume with the injection device, so as to reduce an amount of medicament that is wasted when performing an injection. As still another example, it is desirable for a stopper to have a construction or configuration that reduces stickiness of the stopper within the barrel.

Accordingly, it is desirable to provide a stopper constructed to address the design and use considerations referenced above.

### SUMMARY OF THE INVENTION

Provided herein is a stopper for use in an injection device and adapted for attachment with a plunger rod thereof. The stopper includes a main body defining a proximal end and a distal end in a longitudinal direction of the stopper, the proximal end configured to mate with a distal end of the plunger rod, to secure the stopper to the plunger rod. The stopper also includes a plurality of annular ribs each extending radially outward from an outer surface of the main body and around an outer circumference of the main body, the plurality of annular ribs including at least a first annular rib and a second annular rib spaced from the first annular rib in the longitudinal direction, with an inter-rib region positioned between the first annular rib and the second annular rib. The stopper further includes a plurality of vertical ribs positioned in the inter-rib region and each extending outward from the outer surface of the main body, with the plurality of vertical ribs oriented in the longitudinal direction and spaced about the outer circumference of the main body, the plurality of vertical ribs increasing a rigidity of the stopper in the longitudinal direction.

In certain configurations, the inter-rib region comprises a curved inter-rib region where the outer surface of the main body is curved radially inward, and wherein each of the plurality of vertical ribs has a curved profile matching the curved inter-rib region.

In certain configurations, with the curved profile, each of the plurality of vertical ribs is configured to flatten when the stopper is compressed in the longitudinal direction.

In certain configurations, the plurality of annular ribs further comprises a third annular rib spaced from the second annular rib, the third annular rib extending radially outward from the outer surface of the main body and around the outer circumference of the main body, with a second inter-rib region positioned between the second annular rib and the third annular rib. The stopper further includes a second plurality of vertical ribs positioned in the second inter-rib region and each extending outward from the outer surface of the main body, with the second plurality of vertical ribs oriented in the longitudinal direction and spaced about the outer circumference of the main body.

In certain configurations, the plurality of vertical ribs is integrally formed with the main body.

In certain configurations, the stopper is free of per- and poly- fluoroalkyl substances or contains 200 parts-per-million or less of per- and poly- fluoroalkyl substances, and preferably 50 parts-per-million or less of per- and poly- fluoroalkyl substances.

Also provided herein is a stopper for use in an injection device and adapted for attachment with a plunger rod thereof movable within a barrel of the injection device. The stopper includes a main body defining a proximal end and a distal end in a longitudinal direction of the stopper, the proximal end configured to mate with a distal end of the plunger rod, to secure the stopper to the plunger rod. The stopper also includes a plurality of annular ribs each extending radially outward from an outer surface of the main body and around an outer circumference of the main body, the plurality of annular ribs including at least a first annular rib and a second annular rib spaced from the first annular rib in the longitudinal direction, with an inter-rib region positioned between the first annular rib and the second annular rib. The distal end of the main body comprises a roof portion covering the main body to form a closed distal end, the roof portion comprising a conically-shaped distal-facing surface that includes a pattern of projections and/or grooves formed thereon.

In certain configurations, the conically-shaped distal-facing surface is configured to reduce a dead volume between the distal-facing surface of the stopper and an inner surface of the barrel, when the stopper is fully advanced distally within the barrel.

In certain configurations, the pattern of projections and/or grooves comprises a star-shaped arrangement of projections extending out from a center point of the roof portion.

In certain configurations, the pattern of projections and/or grooves comprises a star-shaped arrangement of grooves extending out from a center point of the roof portion.

In certain configurations, the pattern of projections and/or grooves comprises a plurality of concentric annular projections, with annular grooves formed between each adjacent pair of concentric annular projections.

In certain configurations, the pattern of projections and/or grooves further comprises an arrangement of linear grooves extending out from a center point of the roof portion and bisecting the annular grooves, with grooves of the arrangement of three linear grooves each spaced apart by 120 degrees.

In certain configurations, the pattern of projections and/or grooves comprises a spiraled projection and corresponding spiral groove.

In certain configurations, the arrangement of linear grooves or the spiral groove is configured to generate a fluid flow that aids in expelling fluid out from the barrel as the stopper is advanced distally within the barrel, and to reduce stickiness between the stopper and the barrel.

Also provided herein is a stopper for use in an injection device and adapted for attachment with a plunger rod thereof movable within a barrel of the injection device. The stopper includes a main body defining a proximal end and a distal end in a longitudinal direction of the stopper, the proximal end comprising an open proximal end configured to mate with a distal end of the plunger rod, to secure the stopper to the plunger rod. The stopper also includes a plurality of annular ribs each extending radially outward from an outer surface of the main body and around an outer circumference of the main body, the plurality of annular ribs including at least a first annular rib and a second annular rib spaced from the first annular rib in the longitudinal direction, with an inter-rib region positioned between the first annular rib and the second annular rib. The proximal end of the main body comprises a proximally-facing shoulder portion that includes a pattern of projections formed thereon and spaced circumferentially about the shoulder portion.

In certain configurations, the pattern of projections comprises a plurality of arc-shaped projections formed on the shoulder portion and extending out proximally therefrom.

In certain configurations, the pattern of projections comprises a plurality of wedge-shaped projections formed on the shoulder portion and extending out proximally therefrom.

In certain configurations, the pattern of projections comprises a continuous circular arrangement of interspersed bumps and depressions formed on the shoulder portion.

Also provided herein is a stopper for use in an injection device and adapted for attachment with a plunger rod thereof movable within a barrel of the injection device. The stopper includes a main body defining a proximal end and a distal end in a longitudinal direction of the stopper, the proximal end comprising an open proximal end configured to mate with a distal end of the plunger rod, to secure the stopper to the plunger rod. The stopper also includes at least one annular lip extending radially outward from an outer surface of the main body and around an outer circumference of the main body, so as to interact with the barrel to form a seal therewith. The at least one annular lip comprises a free outer radial edge configured to deflect responsive to movement of the stopper within the barrel, with the annular lip angled in a proximal direction or a distal direction.

In certain configurations, the at least one annular lip is angled in the proximal direction.

In certain configurations, the at least one annular lip is angled in the distal direction.

In certain configurations, the at least one annular lip comprises a distal lip positioned at the distal end of the main body.

In certain configurations, the at least one annular lip further comprises a proximal lip positioned adjacent the proximal end of the main body.

In certain configurations, the stopper also includes an annular rib extending radially outward from an outer surface of the main body and around an outer circumference of the main body.

In certain configurations, the stopper also includes a plurality of vertical ribs positioned each extending outward from the outer surface of the main body, with the plurality of vertical ribs oriented in a longitudinal direction and spaced about the outer circumference of the main body, the plurality of vertical ribs increasing a rigidity of the stopper in the longitudinal direction.

### BRIEF DESCRIPTION OF THE DRAWINGS

FIG. 1 is a perspective view of a syringe, with which embodiments of the disclosure may be implemented;
FIG. 2 is an exploded view of the syringe of FIG. 1;
FIG. 3 is a side view of a stopper that may be included in the syringe of FIG. 1;
FIG. 4 is a side cross-sectional view of the stopper of FIG. 3;
FIG. 5 is a side view of a stopper that may be included in the syringe of FIG. 1; and
FIG. 6 is a side view of a stopper having vertical ribs that may be included in the syringe of FIG. 1, according to an embodiment of the disclosure;
FIG. 7 is a side view of a stopper having vertical ribs that may be included in the syringe of FIG. 1, according to an embodiment of the disclosure;
FIG. 8 is a side view of a stopper having vertical ribs that may be included in the syringe of FIG. 1, according to an embodiment of the disclosure;
FIG. 9 is a side view of a stopper having vertical ribs that may be included in the syringe of FIG. 1, according to an embodiment of the disclosure;
FIG. 10 is a side view of a stopper having vertical ribs that may be included in the syringe of FIG. 1, according to an embodiment of the disclosure;
FIG. 11 is a partial perspective view of a stopper having a patterned roof portion that may be included in the syringe of FIG. 1, according to an embodiment of the disclosure;
FIG. 12 is a partial perspective view of a stopper having a patterned roof portion that may be included in the syringe of FIG. 1, according to an embodiment of the disclosure;
FIG. 13 is a partial perspective view of a stopper having a patterned roof portion that may be included in the syringe of FIG. 1, according to an embodiment of the disclosure;
FIG. 14 is a partial perspective view of a stopper having a patterned roof portion that may be included in the syringe of FIG. 1, according to an embodiment of the disclosure;
FIG. 15 is a partial perspective view of a stopper having a patterned roof portion that may be included in the syringe of FIG. 1, according to an embodiment of the disclosure;
FIG. 16 is a partial perspective view of a stopper having a proximal shoulder with projections thereon that may be included in the syringe of FIG. 1, according to an embodiment of the disclosure;
FIG. 17 is a partial perspective view of a stopper having a proximal shoulder with projections thereon that may be included in the syringe of FIG. 1, according to an embodiment of the disclosure;
FIG. 18 is a partial perspective view of a stopper having a proximal shoulder with projections thereon that may be included in the syringe of FIG. 1, according to an embodiment of the disclosure;
FIG. 19 is a partial perspective view of a stopper having a proximal shoulder with projections thereon that may be included in the syringe of FIG. 1, according to an embodiment of the disclosure;
FIG. 20 is a partial perspective view of a stopper having a proximal shoulder with projections thereon that may be included in the syringe of FIG. 1, according to an embodiment of the disclosure;
FIG. 21 is a side view of a stopper having a proximally angled distal lip that may be included in the syringe of FIG. 1, according to an embodiment of the disclosure;
FIG. 22 is a side cross-sectional view of the stopper of FIG. 21;
FIG. 23 is a side view of a stopper having a proximally angled distal lip and vertical ribs that may be included in the syringe of FIG. 1, according to an embodiment of the disclosure;
FIG. 24 is a side view of a stopper having proximally angled distal and proximal lips that may be included in the syringe of FIG. 1, according to an embodiment of the disclosure;
FIG. 25 is a side cross-sectional view of the stopper of FIG. 24;
FIG. 26 is a side view of a stopper having proximally angled distal and proximal lips and vertical ribs that may be included in the syringe of FIG. 1, according to an embodiment of the disclosure;
FIG. 27 is a side view of a stopper having a proximally angled distal lip and a pair of annular ribs that may be included in the syringe of FIG. 1, according to an embodiment of the disclosure;
FIG. 28 is a side view of a stopper having a distally angled distal lip and a pair of annular ribs that may be included in the syringe of FIG. 1, according to an embodiment of the disclosure;
FIG. 29 is a side cross-sectional view of the stopper of FIG. 28;
FIG. 30 is a side view of a stopper having a distally angled distal and proximal lips and an annular rib that may be included in the syringe of FIG. 1, according to an embodiment of the disclosure;
FIG. 31 is a side cross-sectional view of the stopper of FIG. 30;
FIG. 32 is a side view of a stopper having a distally angled proximal lip and a pair of annular ribs that may be included in the syringe of FIG. 1, according to an embodiment of the disclosure;

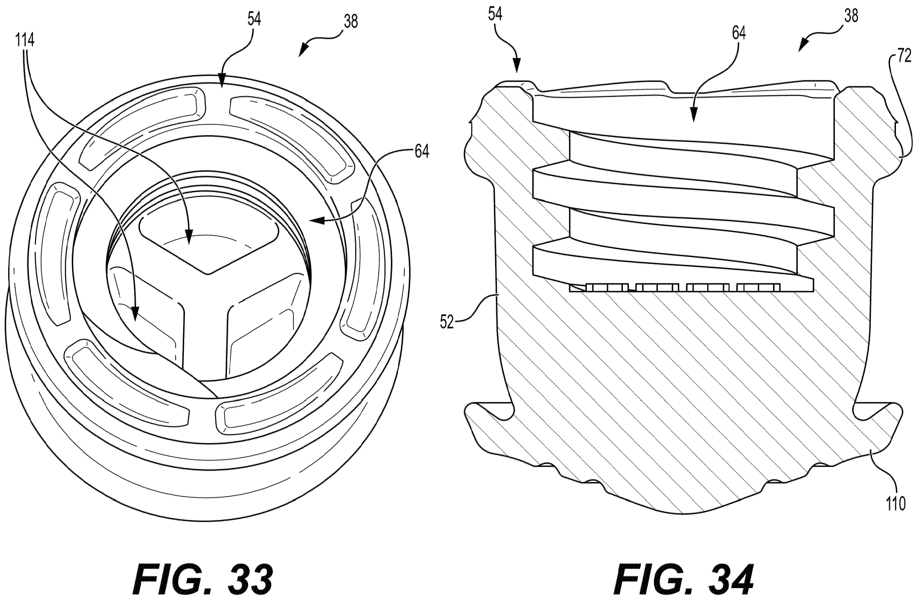

FIG. 33 is a perspective view of a reduced weight stopper that may be included in the syringe of FIG. 1, according to an embodiment of the disclosure; and
FIG. 34 is a side cross-sectional view of the stopper of FIG. 33.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

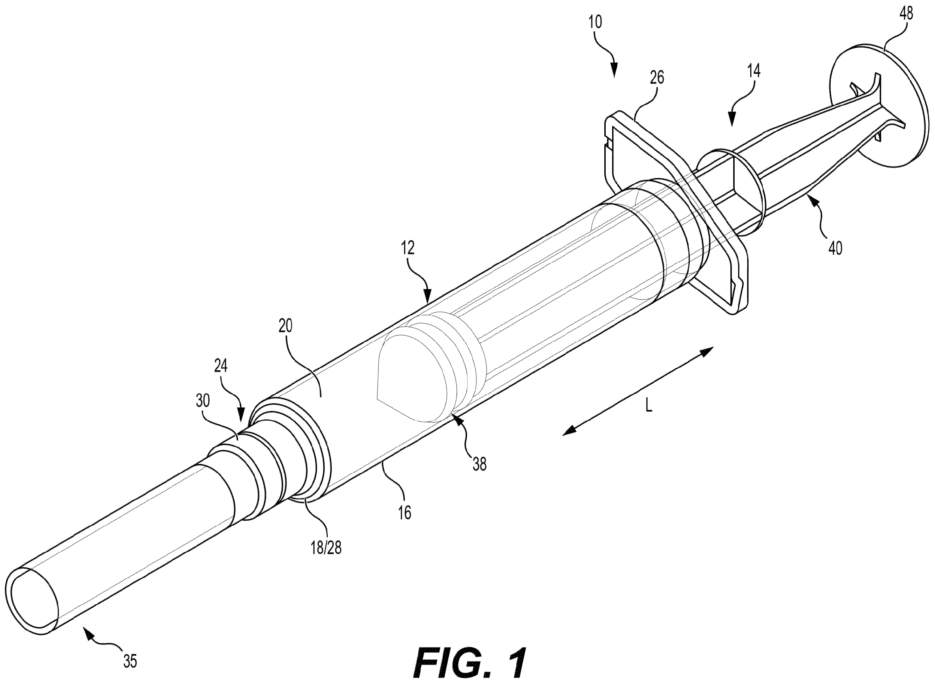

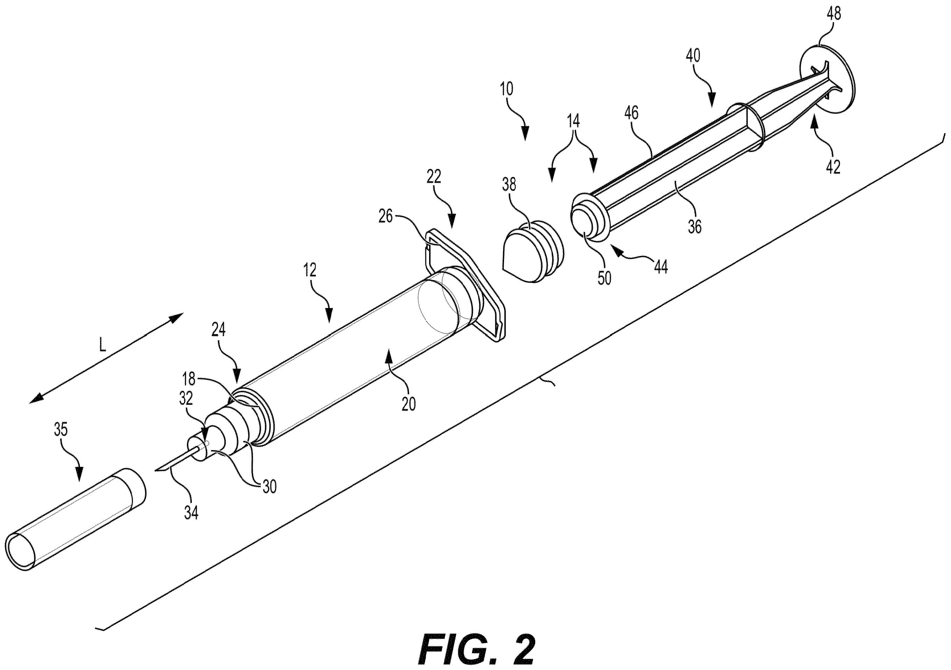

Referring to FIGS. 1 and 2, shown is a non-limiting embodiment of an injection device 10 with which aspects or embodiments of the disclosure may be implemented. According to some aspects of the disclosure, the injection device 10 is provided as a prefilled syringe (hereafter, "syringe 10"), which provides the convenience of rapidly delivering the liquid therein to a patient without the need to first aspirate the medication from another container and meter its volume. According to other aspects of the disclosure, the injection device 10 could instead be provided as an auto-injector, with it recognized that embodiments of the disclosure could alternatively be implemented in such an auto-injector rather than a syringe.

As shown in FIGS. 1 and 2, a syringe 10 generally includes a syringe barrel 12 and a plunger assembly 14. The plunger assembly 14 is movable within the syringe barrel 12 along a longitudinal axis or direction L to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, an extension member 50 (e.g., threaded cylindrical member) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper 38, with the protrusion and receptacle engaging via threading, for example.

The stopper 38 of plunger assembly 14 is positioned at the plunger distal end 44 so as to be movable along with the plunger 36 within the chamber 20 of syringe barrel 12. That is, as a distally-directed force is applied to the plunger rod 36 (such as to thumb press 48), both the plunger rod 36 and the stopper 38 are caused to move distally through syringe barrel 12, with the stopper 38 configured to maintain a seal with an inner surface of the syringe barrel 12 as it is moved therethrough.

According to aspects of the disclosure, the stopper 38 may be made from a material that is different from the material of the plunger 36 and that is capable of forming a tight seal with the syringe barrel 12 as it is advanced therethrough. In some embodiments, the stopper 38 is formed of a compliant or an elastomeric material such as butyl, styrene butadiene, isoprene, or liquid silicone rubber (LSR), as non-limiting examples. In other embodiments, the stopper 38 may instead be formed of a polymeric material, such as cross-linked or thermoplastic elastomers or other thermoplastic materials (e.g., TPS, TPV, etc.), as non-limiting examples. In some exemplary embodiments, whether the stopper 38 is formed of an elastomeric material or a polymeric material, the stopper is formed to be free or substantially free of per- and poly- fluoroalkyl substances (PFAS) - generally, a "PFAS-free" material - that might leach into the medicament or other solution stored in pre-filled syringe 10. As defined herein, the stopper 38 being "free or substantially free of PFAS" or "PFAS-free" is understood to refer to materials that are entirely free of PFAS and/or materials that may contain trace amounts of PFAS therein. In an exemplary embodiment, the stopper 38 is considered to be substantially PFAS-free if it contains 400 arts-per-million (PPM) or less of PFAS therein (i.e., no more than 400 PPM), preferably 200 PPM or less of PFAS therein, and even more preferably 50 PPM or less of PFAS therein.

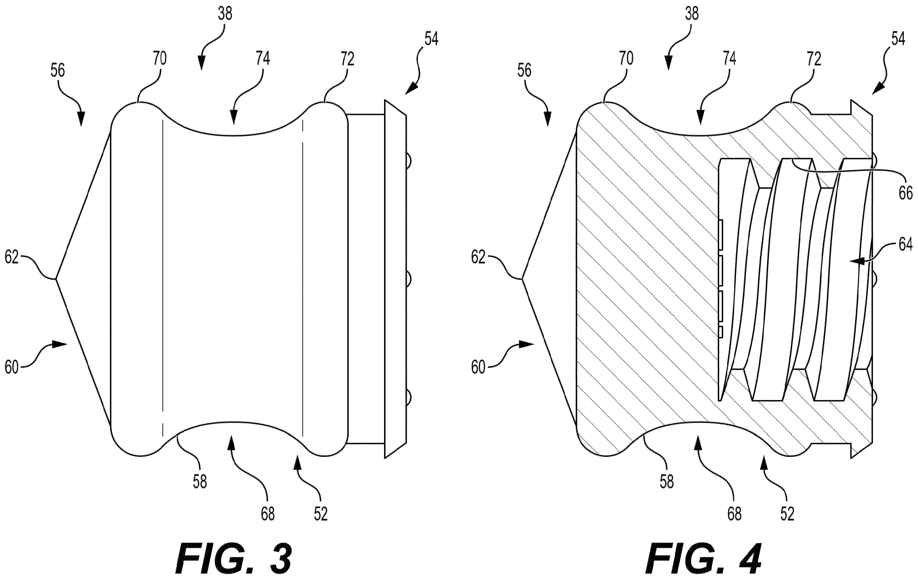

Referring now to FIGS. 3 and 4, and with continued reference to FIGS. 1 and 2, the structure of stopper 38 is shown in greater detail, according to an aspect or embodiment of the disclosure. The stopper 38 is defined by a main body 52 that includes an open "tail" or proximal end 54 that engages with the distal end 44 of plunger 36 and a closed "head" or distal end 56 configured to engage with the barrel 12 of syringe 10. The closed distal end 56 of the main body 52 includes a generally cylindrical portion 58 and a head or roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, as explained in further detail below.

As shown in FIG. 4, the main body 52 of the stopper 38 is partially hollow and is sized and configured to receive the extension member 50 of plunger 36 therein. The main body 52 of the stopper 38 defines an inner cavity 64 that is generally defined between a majority of the open proximal end 54 and the closed distal end 56 of the main body 52. In some embodiments, and as previously indicated, the inner cavity 64 includes a threaded inner surface 66 that is configured to receive and mate with a threaded extension member 50 of plunger 36. In other embodiments, the inner cavity 64 may define an inner contoured surface having a notch therein that is configured to receive a flanged extension member of plunger 36, with the flanged extension member engaging the notch in order to retain the extension member 50 within the inner cavity 64.

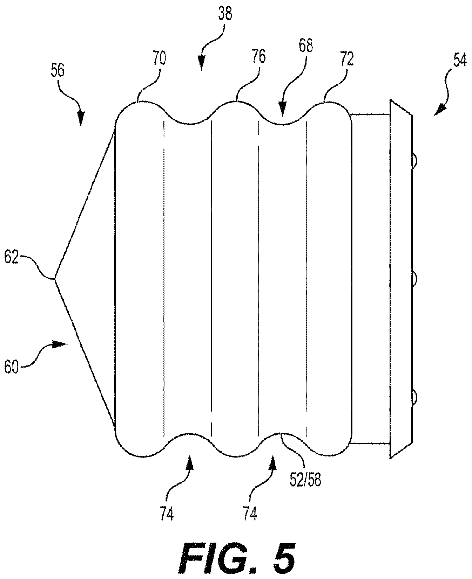

Referring still to FIGS. 3 and 4, the outer surface 68 of the cylindrical portion 58 of the main body 52 includes a plurality of annular ribs 70, 72 formed thereon that extend circumferentially around the entire outer surface 68 of the cylindrical portion 58. In the illustrated embodiment, the plurality of annular ribs 70, 72 includes a first annular rib 70 positioned toward the proximal end 54 of the main body 52 and a second annular rib 72 positioned toward the distal end 56 of the main body 52 - with the first annular rib 70 spaced apart longitudinally from the second annular rib 72 on the cylindrical portion 58 to form an inter-rib region 74 therebetween. While the stopper 38 is shown in FIGS. 3 and 4 as including only two (2) annular ribs 70, 72 formed thereon (i.e., first annular rib 70 and second annular rib 72), it is recognized that stopper 38 may be constructed to include a greater number of annular ribs formed thereon, such as three (3) annular ribs thereon, with a third annular rib 76 illustrated in FIG. 5, with an inter-rib region 74 provided between each adjacent pair of annular ribs.

According to aspects of the disclosure, the inter-rib region 74 positioned between the first annular rib 70 and the second annular rib 72 (and between any other adjacent pair of annular ribs, if there are three or more annular ribs) is formed to provide a curved outer surface 68 on a section/region of the cylindrical portion 58 of the main body 52. The inter-rib region 74 curves radially inward, such that a maximum outer diameter of the inter-rib region 74 is provided adjacent annular ribs 70, 72 and a minimum outer diameter of the inter-rib region 74 is provided at a longitudinal mid-point of the inter-rib region 74.

According to one aspect of the disclosure, stopper 38 may be structured to increase the stopper rigidity in the longitudinal direction L, to provide for better force transmission applied to the stopper 38 and to limit radial deformation of the stopper 38, thereby optimizing the stopper 38 for better behavior during an auto-injector activation phase, better resistance during stoppering, and better avoidance of stopper tilting during stoppering, while maintaining optimal gliding and leak performance.

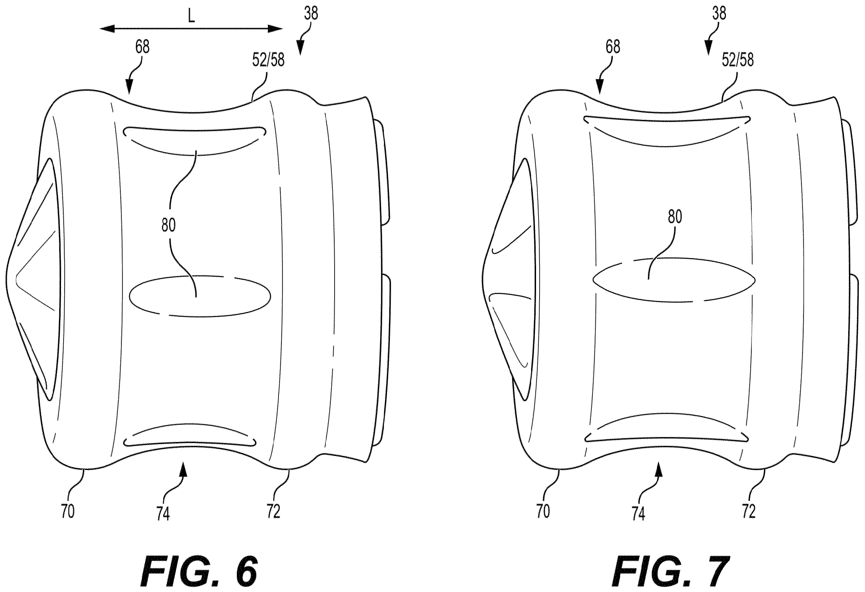

Referring now to FIGS. 6-10, various embodiments of a stopper 38 having an increased rigidity in/along the longitudinal direction L are illustrated. The structure of each of these stopper embodiments are generally similar to the stopper 38 shown and described in FIGS. 3 and 4, except that additional stiffening structures are added to the stopper. Specifically, a plurality of vertical ribs 80 is provided on stopper 38 that increase a rigidity of the stopper 38. The vertical ribs 80 are oriented in the longitudinal direction L and are positioned in the inter-rib region(s) 74 of stopper 38 and, with the vertical ribs 80 spaced about the outer circumference of the main body 52 in the inter-rib region(s) 74. The vertical ribs 80 extend outward from the outer surface 68 of the cylindrical portion 58 of the main body 52, but extend radially outward from the main body 52 by an amount less than the annular ribs 70, 72, 76, so as to not interfere with the sealing capabilities of annular ribs 70, 72, 76. In some embodiments, the vertical ribs 80 are integrally formed with the main body 52, such as via forming of the stopper 38 using a molding process.

In some embodiments, the inter-rib region 74 comprises a curved inter-rib region where the outer surface 68 of the cylindrical portion 58 of the main body 52 is curved radially inward. In such embodiments, each of the vertical ribs 80 may have a curved profile that generally matches the curved profile of the inter-rib region 74. With the vertical ribs 80 having a curved profile, the vertical ribs 80 may be configured to flatten/straighten when the stopper 38 is compressed in the longitudinal direction L, so as to increase the stopper rigidity in/along the longitudinal direction L.

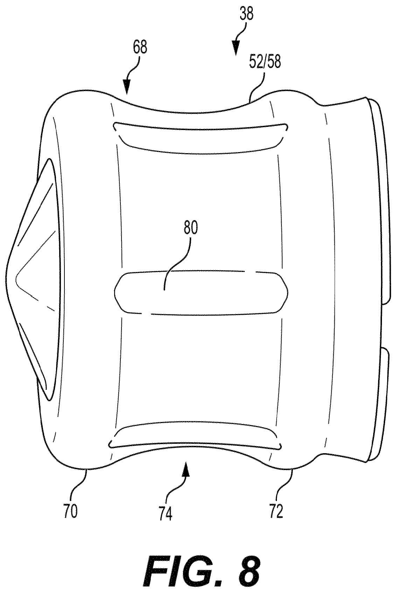

FIGS. 6-8 illustrate various stoppers 38 that each include a first annular rib 70 and a second annular rib 72, with vertical ribs 80 provided in the inter-rib region 74 between the first annular rib 70 and the second annular rib 72. In the embodiment of FIG. 6, the vertical ribs 80 extend longitudinally along only a partial length of the inter-rib region 74, with the vertical ribs 80 thus spaced apart longitudinally from the annular ribs 70, 72. In the embodiments of FIGS. 7 and 8, the vertical ribs 80 extend longitudinally along an entirety of the length of the inter-rib region 74, with opposing ends of the vertical ribs 80 thus intersecting with the annular ribs 70, 72.

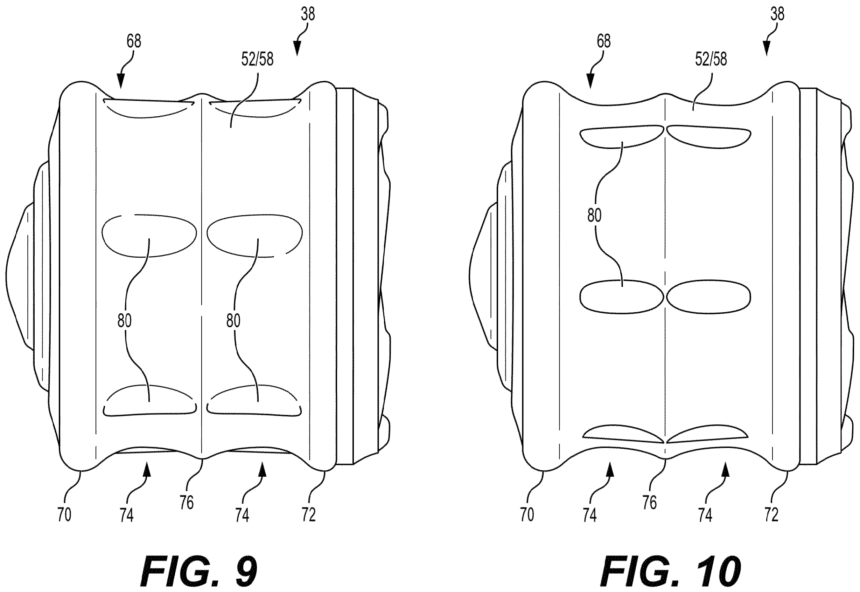

FIGS. 9 and 10 illustrate various stoppers 38 that each include a first annular rib 70, a second annular rib 72, and a third annular rib 76, with vertical ribs 80 provided in the inter-rib region 74 between the first annular rib 70 and the third annular rib 76 and vertical ribs 80 provided in the inter-rib region 74 between the second annular rib 72 and the third annular rib 76 (i.e., a first plurality of vertical ribs 80 between the first and third annular ribs 70, 76 and a second plurality of vertical ribs 80 between the second and third annular ribs 72, 76. In the embodiment of FIG. 9, the vertical ribs 80 in each inter-rib region 74 extend longitudinally along only a partial length of the inter-rib region 74, with the vertical ribs 80 thus joined with/intersecting the third annular rib 76 but spaced apart longitudinally from the annular first and second ribs 70, 72. In the embodiments of FIG. 10, the vertical ribs 80 in each inter-rib region 74 extend longitudinally along an entirety of the length of their respective inter-rib region 74, with the vertical ribs 80 thus joined with/intersecting the third annular rib 76 and one of the first annular rib 70 and the second annular rib 72.

According to another aspect of the disclosure, stopper 38 may be structured to improve injection of a fluid out from the injection device 10, such as by reducing a dead volume between the distal-facing surface of the stopper 38 and an inner surface of the barrel 12 when the stopper 38 is fully advanced distally within the barrel 12 and/or by generating a fluid flow that better expels fluid out from the barrel 12 as the stopper 38 is advanced distally within the barrel 12. The stopper 38 may be further structured to reduce stickiness thereof within the barrel 12.

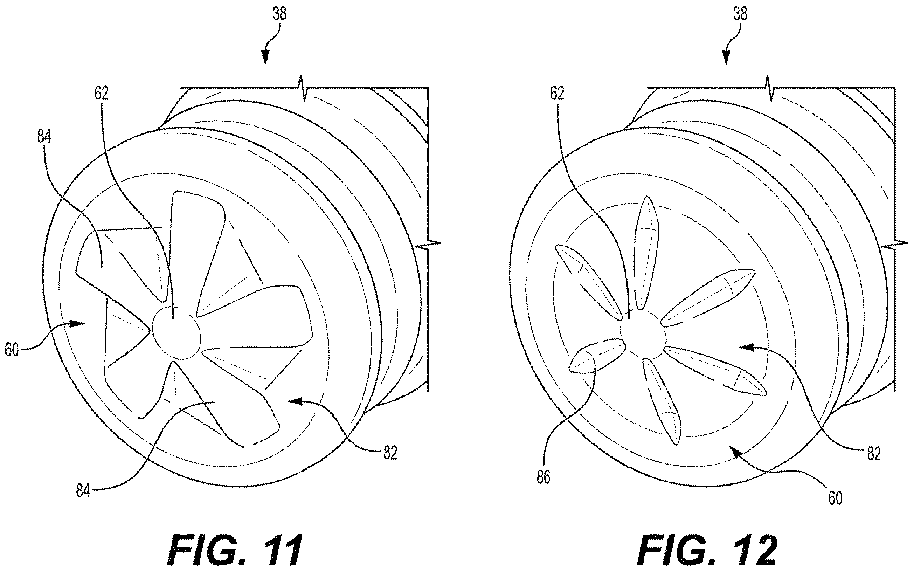

Referring now to FIGS. 11-15, various embodiments of a stopper 38 are illustrated by which a dead volume may be reduced and/or by which improved fluid injection may be realized. The structure of each of these stopper embodiments are generally similar to the stopper 38 shown and described in FIGS. 3 and 4, except that additional features are provided on the roof portion 60. Specifically, the roof portion 60 comprises a conically-shaped distal-facing surface 82 that includes a pattern of projections and/or grooves formed thereon. The projections and/or grooves are formed on the conically-shaped distal-facing surface 82 to better interfit with the inner surface of the barrel 12 at the distal end 24 (to reduce the dead volume) and/or to create a fluid flow that aids is expelling fluid from the barrel 12 during an injection.

FIG. 11 illustrates a stopper 38 that includes a pattern of projections 84 that comprises a star-shaped arrangement of projections 84 extending out from a center point (i.e., tip 62) of the roof portion 60. The star-shaped arrangement of projections 84 projects out from a remainder of the conically-shaped distal-facing surface 82 of roof portion 60, so as to better interfit with the inner surface of the barrel 12 at the distal end 24 and reduce the dead volume in the syringe 10.

FIG. 12 illustrates a stopper 38 that includes a pattern of grooves 86 that comprises a star-shaped arrangement of grooves 86 extending out from a center point (i.e., tip 62) of the roof portion 60. The star-shaped arrangement of grooves 86 is formed into the conically-shaped distal-facing surface 82 of roof portion 60 and acts to create a fluid flow that aids is expelling fluid from the barrel 12 during an injection from syringe 10.

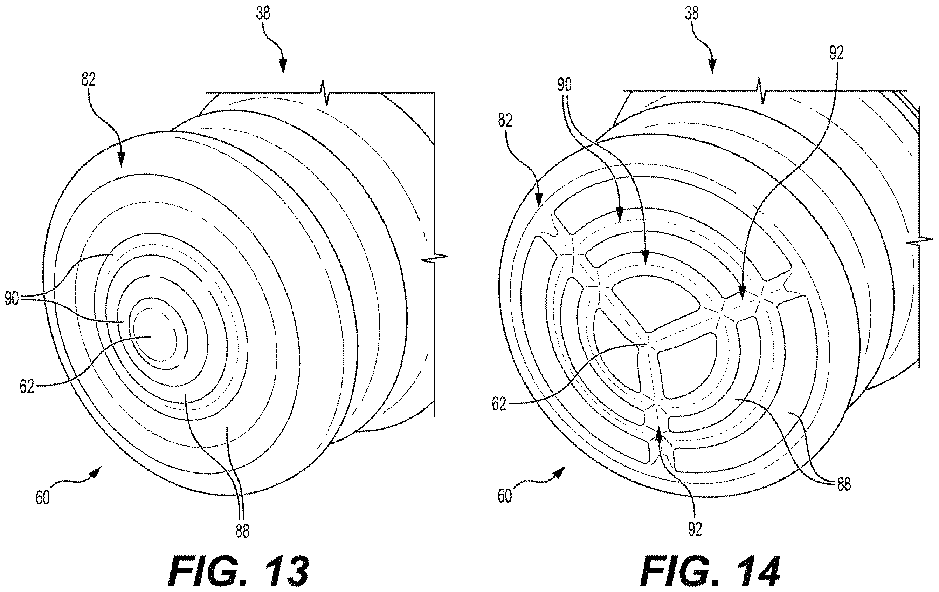

FIG. 13 illustrates a stopper 38 that includes a pattern of projections and grooves that comprises a plurality of concentric annular projections 88, with annular grooves 90 formed between each adjacent pair of concentric annular projections 88. The concentric annular projections 88 and associated annular grooves 90 is formed on the conically-shaped distal-facing surface 82 of roof portion 60 and is configured to better interfit with the inner surface of the barrel 12 at the distal end 24 and reduce the dead volume in the syringe 10.

FIG. 14 illustrates a stopper 38 that includes a pattern of projections and grooves that comprises a plurality of concentric annular projections 88, with annular grooves 90 formed between each adjacent pair of concentric annular projections 88, with an additional arrangement of three linear grooves 92 extending out from a center point (i.e., tip 62) of the roof portion 60 that bisects the annular projections 88 and grooves 90. The arrangement of three linear grooves 92 is configured such that the linear grooves 92 spaced apart from one another by 120 degrees. This configuration of linear grooves 92 (along with the concentric annular projections/grooves 88, 90) aids is expelling fluid from the barrel 12 during an injection from syringe 10.

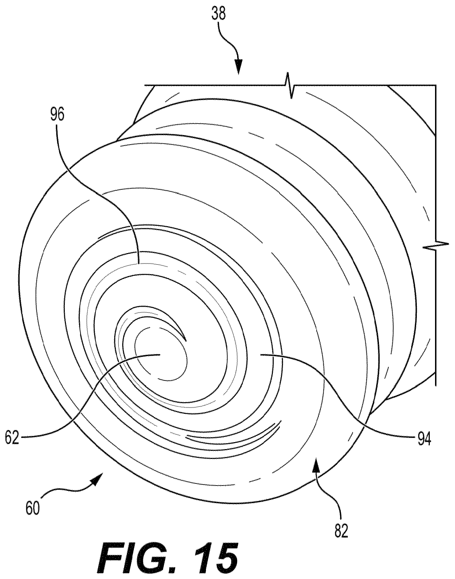

FIG. 15 illustrates a stopper 38 that includes a pattern of projections and grooves that comprises a spiraled projection 94 and corresponding spiral groove 96. The spiraled projection 94 and corresponding spiral groove 96 aids is expelling fluid from the barrel 12 during an injection from syringe 10.

According to another aspect of the disclosure, stopper 38 may be structured to reduce stickiness of the stopper 38 and/or to help the stopper 38 better resist deformation during advancement thereof.

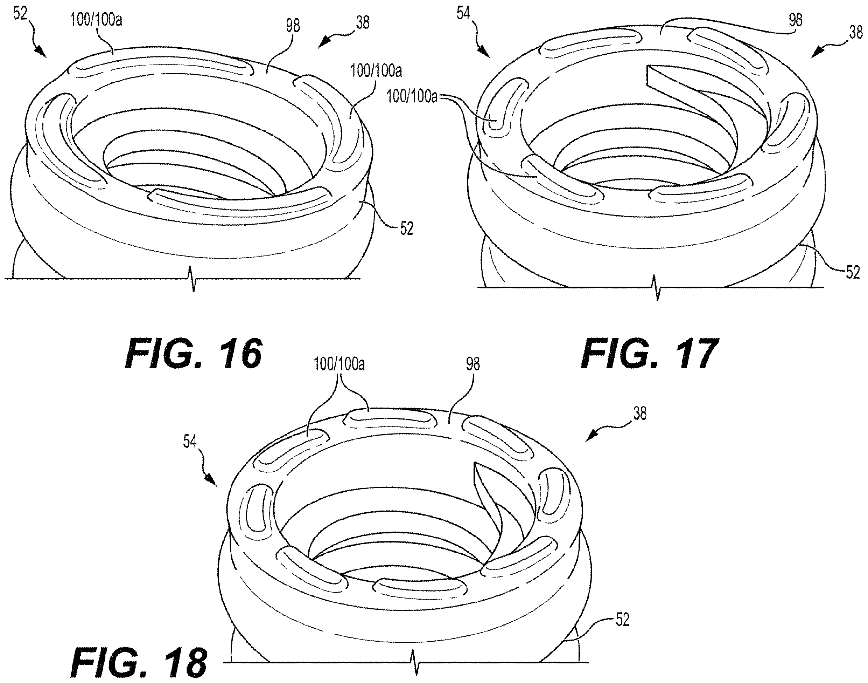

Referring now to FIGS. 16-20, various embodiments of a stopper 38 are illustrated by which stickiness of the stopper 38 may be reduced and/or by which stopper deformation may be reduced during advancement thereof. The structure of each of these stopper embodiments are generally similar to the stopper 38 shown and described in FIGS. 3 and 4, except that additional features are provided on the proximal end 54 of the main body 52 of stopper 38. Specifically, a proximally-facing shoulder portion 98 of the main body 52 is formed to include a pattern of projections 100 formed thereon and spaced circumferentially about the shoulder portion 98. The pattern of projections 100 reduced stickiness of the stopper 38 and that help the stopper 38 reduce deformation during distal advancement through the barrel 12.

According to one aspect, the projections 100 on the proximally-facing shoulder portion 98 provide an anti-stick tail on the stopper 38, with the projections 100 reducing a contact surface area between the stopper 38 and a plunger rod 36 that contacts the stopper 38, to reduce the "stickiness" of the stopper 38.

According to another aspect, the projections 100 on the proximally-facing shoulder portion 98 help to the increase the robustness of the stopper 38 when a pressing force is applied thereto, such that the stopper 38 may better resist unexpected deformation. In one embodiment, the projections 100 on the proximally-facing shoulder portion 98 may help to the increase the robustness of the stopper 38 when integrated into an auto-injector, when the stopper 38 is advanced during an injection via the auto-injector .

FIGS. 16-18 illustrate a stopper 38 that includes a plurality of arc-shaped projections 100a formed on the shoulder portion 98 and extending out proximally therefrom. The arc-shaped projections 100a are spaced circumferentially about the shoulder portion 98, with each of the projections 100a extending over an angular range of the shoulder portion 98. The number of arc-shaped projections 100a formed on the shoulder portion 98 may vary, with a series of four projections (FIG. 16), six projections (FIG. 17), or eight projections (FIG. 18) being non-limiting examples.

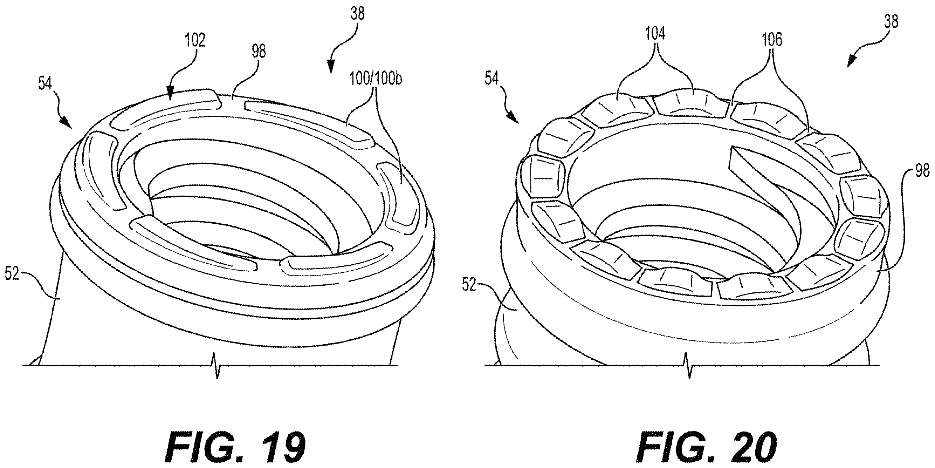

FIG. 19 illustrates a stopper 38 that includes a plurality of wedge-shaped projections 100b formed on the shoulder portion 98 and extending out proximally therefrom. The arc-shaped projections 100b are spaced circumferentially about the shoulder portion 98, with each of the projections 100b extending over an angular range of the shoulder portion 98. Each of the wedge-shaped projections 100b includes a sloped proximally-facing surface 102 thereon. While the embodiment of FIG. 19 shows an arrangement of six wedge-shaped projections 100b formed on the shoulder portion 98, it is recognized that a greater or lesser number of wedge-shaped projections 100b could be provided on the shoulder portion 98, according to other embodiments.

FIG. 20 illustrates a stopper 38 that includes a continuous circular arrangement of interspersed bumps 104 and depressions 106 formed on the shoulder portion 98. The circular arrangement of interspersed bumps 104 and depressions 106 may structure a proximally-facing surface of the shoulder portion 98 to have a wavy profile. In some embodiments, the number of bumps 104 may be greater than the number of depressions 106 (by one).

According to another aspect of the disclosure, stopper 38 may be structured to improve gliding performance/force of the stopper 38 as it is advanced through barrel 12, while still forming a desired seal with the barrel 12 to maintain container closure integrity. The stopper 38 may further be structured to reduce stickiness of the stopper 38 and limit/reduce a dead volume between the distal-facing surface of the stopper 28 and an inner surface of the barrel 12 when the stopper 38 is fully advanced distally within the barrel 12.

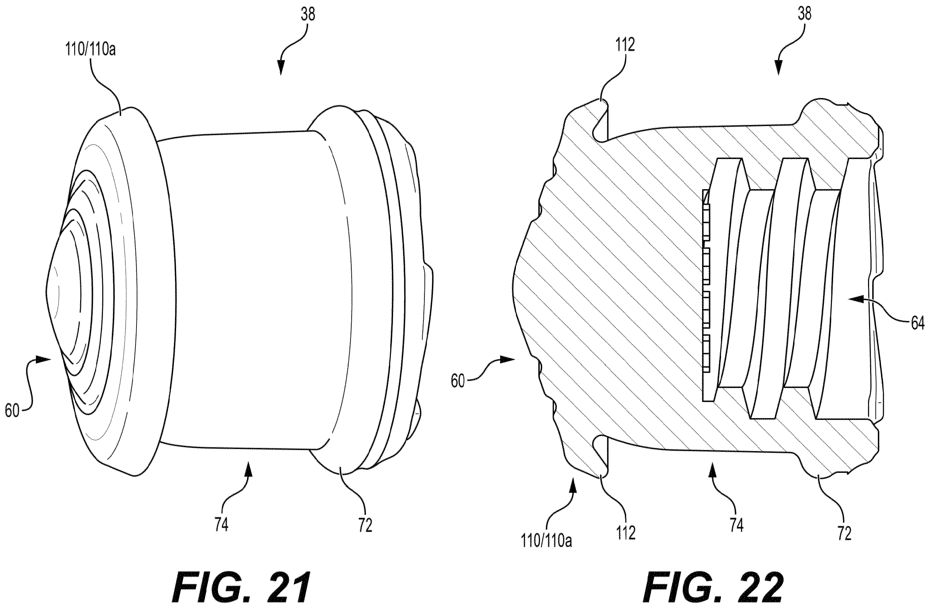

Referring now to FIGS. 21-32, various embodiments of a stopper 38 are illustrated by which gliding performance is improved, stickiness of the stopper 38 within the barrel 12 is reduced, and/or a dead volume in the injection device 10 is reduced. The structure of each of these stopper embodiments are generally similar to the stopper 38 shown and described in FIGS. 3 and 4, except that one or more lips 110 are formed on the stopper 38 that replace one or more of annular ribs 70, 72. Each of the one or more lips 110 is configured as an annular lip that comprises a free outer radial edge 112 configured to deflect responsive to movement of the stopper 38 within the barrel 12. The annular lip(s) 110 may be angled in a proximal direction or a distal direction, to improve the gliding capability of the stopper 38, reduce the stickiness of the stopper 38, and/or reduce a dead volume in the injection device 10.

FIGS. 21 and 22 illustrate a stopper 38 that includes a distal lip 110a that is angled in the proximal direction, along with a second (or proximal) annular rib 72. The combination of the lip 110a and the annular rib 72 functions to ensure proper sealing between the stopper 38 and the barrel 12, while also reducing the gliding force required to advance the stopper 38 within the barrel 12.

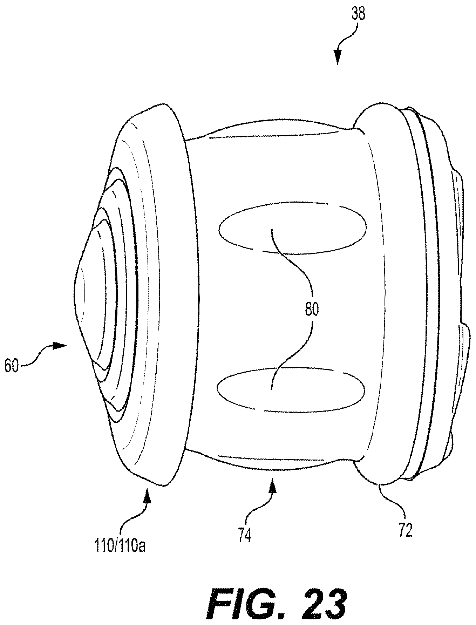

FIG. 23 illustrates another stopper 38 that includes a distal lip 110a that is angled in the proximal direction, along with a second (or proximal) annular rib 72. The stopper 38 is identical to the stopper 38 of FIGS. 21 and 22, except that the stopper 38 further includes a plurality of vertical ribs 80 provided in an "inter-rib region" 74 between the lip 110a and the second annular rib 72. As previously described in the embodiments of FIGS. 6-10, the vertical ribs 80 function to increase a rigidity of the stopper 38 in the longitudinal direction.

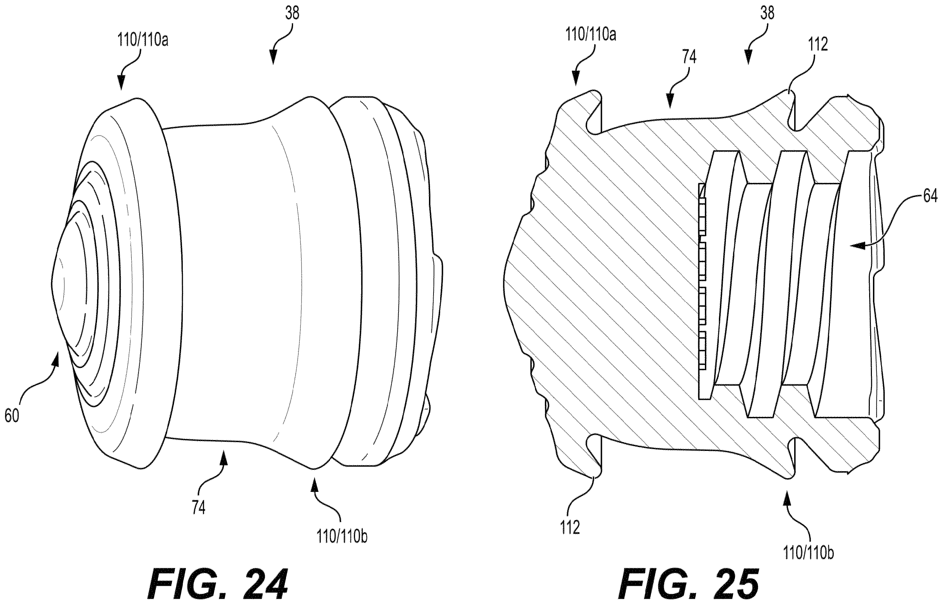

FIGS. 24 and 25 illustrate a stopper 38 that includes both a distal lip 110a and a proximal lip 110b that are angled in the proximal direction. The combination of the distal lip 110a and the proximal lip 110b functions to ensure proper sealing between the stopper 38 and the barrel 12, while also reducing the gliding force required to advance the stopper 38 within the barrel 12.

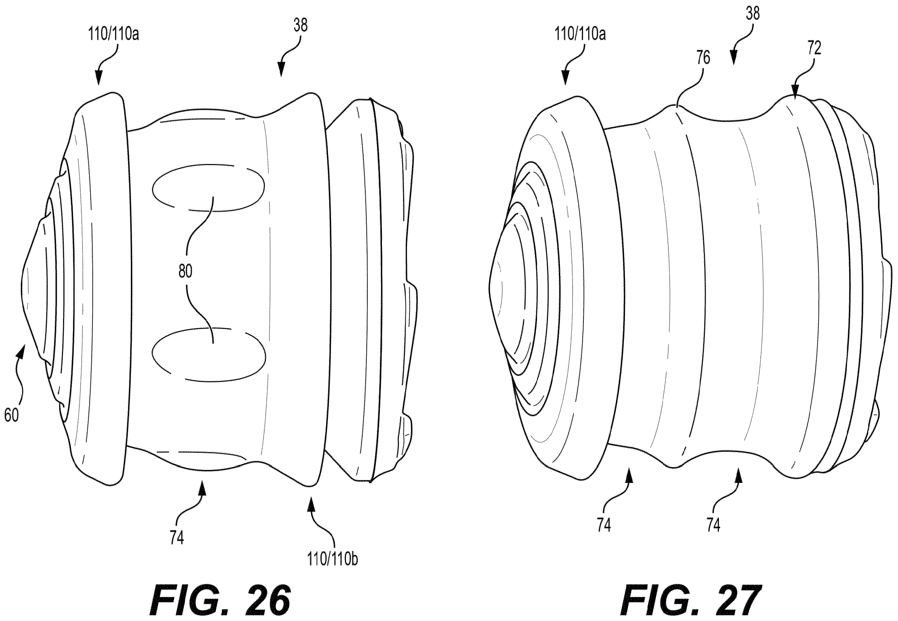

FIG. 26 illustrates another stopper 38 that includes a distal lip 110a and a proximal lip 110b that are angled in the proximal direction. The stopper 38 is identical to the stopper 38 of FIGS. 24 and 25, except that the stopper 38 further includes a plurality of vertical ribs 80 provided in an "inter-rib region" 74 between the distal lip 110a and the proximal lip 110b. As previously described in the embodiments of FIGS. 6-10, the vertical ribs 80 function to increase a rigidity of the stopper 38 in the longitudinal direction.

FIG. 27 illustrates another stopper 38 that includes a distal lip 110a that is angled in the proximal direction, along with a second (or proximal) annular rib 72 and a third annular rib 76. The combination of the distal lip 110a and the second and third annular ribs 72, 76 functions to ensure proper sealing between the stopper 38 and the barrel 12, while also reducing the gliding force required to advance the stopper 38 within the barrel 12.

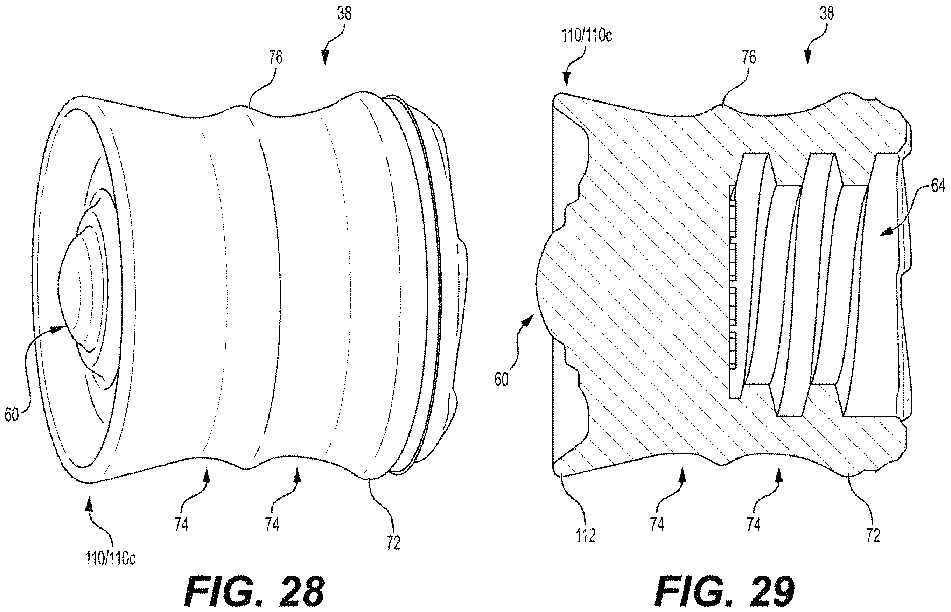

FIGS. 28 and 29 illustrate a stopper 38 that includes a distal lip 110c that is angled in the distal direction, along with a second (or proximal) annular rib 72 and a third annular rib 76. The combination of the lip 110c and the second and third annular ribs 72, 76 functions to ensure proper sealing between the stopper 38 and the barrel 12, while also improving moldability of the stopper 38 (due to the lip 110c being angled in the mold ejection direction) and reducing a dead volume between the distal-facing surface of the stopper 38 and an inner surface of the barrel 12 when the stopper 38 is fully advanced distally within the barrel 12 (due to the lip 110c being deflected/deformed back proximally upon contacting the barrel distal end 24).

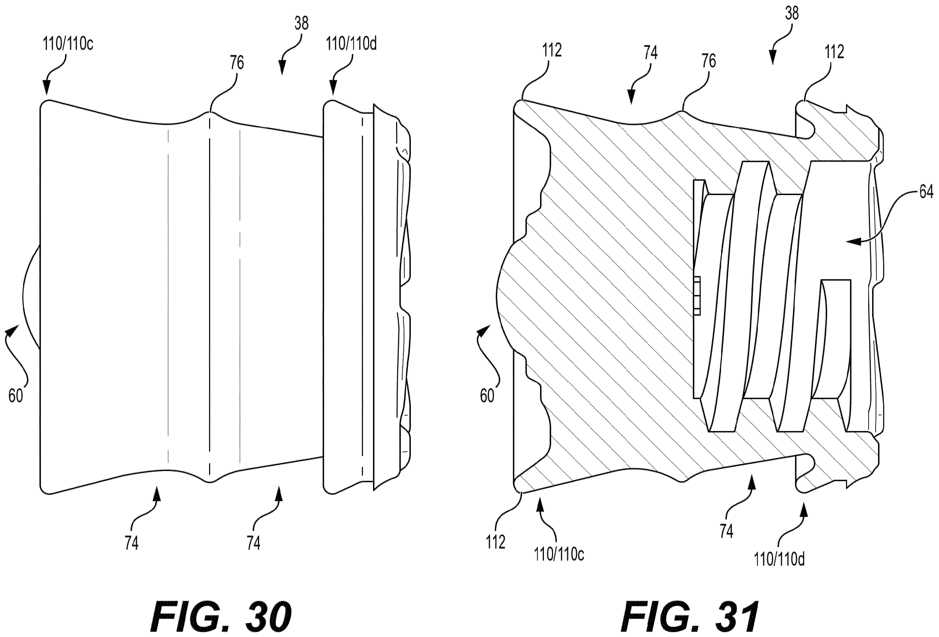

FIGS. 30 and 31 illustrate a stopper 38 that includes both a distal lip 110c and a proximal lip 110d that are angled in the distal direction, along with a third annular rib 76. The combination of the distal lip 110c, the proximal lip 110d, and the third annular rib 76 functions to ensure proper sealing between the stopper 38 and the barrel 12, while also improving moldability of the stopper 38 (due to the lips 110c, 110d being angled in the mold ejection direction) and reducing a dead volume between the distal-facing surface of the stopper 38 and an inner surface of the barrel 12 when the stopper 38 is fully advanced distally within the barrel 12 (due to the distal lip 110c being deflected/deformed back proximally upon contacting the barrel distal end 24).

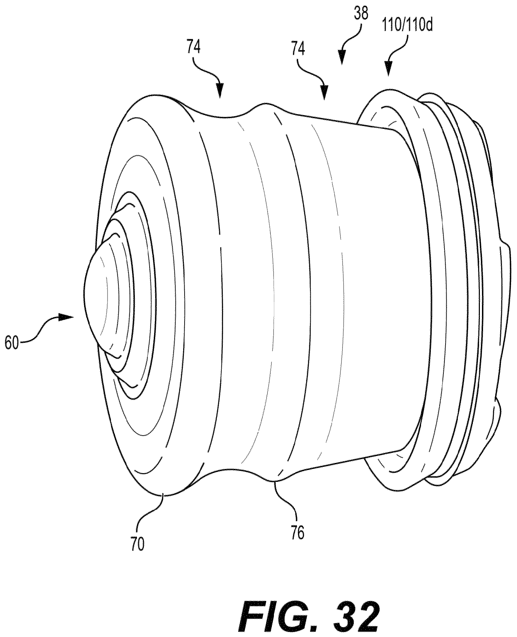

FIG. 32 illustrates a stopper 38 that includes a proximal lip 110d that is angled in the distal direction, along with a first (or distal) annular rib 70 and a third annular rib 76. The combination of the lip 110d and the first and third annular ribs 70, 76 functions to ensure proper sealing between the stopper 38 and the barrel 12, while also improving moldability of the stopper 38 (due to the lip 110d being angled in the mold ejection direction).

According to a further aspect of the disclosure, and for any of the stopper embodiments shown and described in FIGS. 1-32, the stopper 38 may be further modified to reduce the material amount and weight of the stopper 38. Specifically, additional elastomeric material may be removed from stopper 38 in the area of cavity 64. In some embodiments, additional elastomeric material may be removed from stopper 38 by forming pockets 114 at the distal end of the cavity 64. In some embodiments, removal of additional material may reduce the weight of the stopper 38 and facilitate stopper radial deformation during stoppering. In some embodiments, removal of additional material may optimize the tail/proximal end 54 of the stopper 38, such as by eliminating sharp edges on the tail/proximal end 54, thereby reduced stickiness of the stopper 38 and facilitating screwing of the plunger 36 into the stopper 38. In some embodiments, removal of additional material from a distal end of the cavity 64 may help to accommodate the extension member 50 of the plunger 36 (FIG. 2) within the cavity 64, such as when gate vestiges (from a molding process) remain on the extension member 50 of the plunger 36. In some embodiments, a weight reduction of about 0.034 grams may be achieved via removal of additional material from the stopper 38.

Beneficially, embodiments of the invention thus are directed to a stopper for use in an injection device, with the stopper including one or more features thereon that address issues related to container closure integrity, gliding force, stopper deformation, minimization of dead volume in the injection device, and stickiness of the stopper. The stopper may include vertical ribs, a patterned roof portion, a proximal shoulder portion with projections thereon, and/or one or more annular lips, with a combination of one or more of these features addressing the aforementioned issues.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A stopper (38) for use in an injection device (10) and adapted for attachment with a plunger rod (36) thereof, the stopper comprising:
a main body (40, 52) defining a proximal end and a distal end in a longitudinal direction of the stopper, the proximal end configured to mate with a distal end of the plunger rod, to secure the stopper to the plunger rod; and
a plurality of annular ribs (70, 72, 76) each extending radially outward from an outer surface of the main body and around an outer circumference of the main body, the plurality of annular ribs including at least a first annular rib (70) and a second annular rib (72) spaced from the first annular rib in the longitudinal direction, with an inter-rib region positioned between the first annular rib and the second annular rib;
wherein the stopper further includes a plurality of vertical ribs (80) positioned in the inter-rib region and each extending outward from the outer surface of the main body, with the plurality of vertical ribs oriented in the longitudinal direction and spaced about the outer circumference of the main body, the plurality of vertical ribs increasing a rigidity of the stopper in the longitudinal direction.

2. The stopper (38) of claim 1, wherein the inter-rib region comprises a curved inter-rib region where the outer surface of the main body (40, 52) is curved radially inward, each of the plurality of vertical ribs (80) has a curved profile matching the curved inter-rib region, and, optionally, with the curved profile, each of the plurality of vertical ribs (80) is configured to flatten when the stopper is compressed in the longitudinal direction.

3. The stopper (38) of any of claims 1-2, wherein the plurality of annular ribs (70, 72, 76) further comprises a third annular rib (76) spaced from the second annular rib (72), the third annular rib extending radially outward from the outer surface of the main body (40, 52) and around the outer circumference of the main body, with a second inter-rib region positioned between the second annular rib and the third annular rib; and
wherein the stopper further includes a second plurality of vertical ribs (80) positioned in the second inter-rib region and each extending outward from the outer surface of the main body, with the second plurality of vertical ribs oriented in the longitudinal direction and spaced about the outer circumference of the main body.

4. The stopper (38) of any of claims 1-3, wherein the plurality of vertical ribs (80) is integrally formed with the main body (40, 52).

5. The stopper (38) of any of claims 1-4, wherein the stopper is free of per- and poly- fluoroalkyl substances or contains 200 parts-per-million or less of per- and polyfluoroalkyl substances, and preferably 50 parts-per-million or less of per- and poly- fluoroalkyl substances.

6. A stopper (38) for use in an injection device (10) and adapted for attachment with a plunger rod (36) thereof movable within a barrel (12) of the injection device, the stopper comprising:
a main body (40, 52) defining a proximal end and a distal end in a longitudinal direction of the stopper, the proximal end configured to mate with a distal end of the plunger rod, to secure the stopper to the plunger rod; and
a plurality of annular ribs (70, 72, 76) each extending radially outward from an outer surface of the main body and around an outer circumference of the main body, the plurality of annular ribs including at least a first annular rib (70) and a second annular rib (72) spaced from the first annular rib in the longitudinal direction, with an inter-rib region positioned between the first annular rib and the second annular rib;
wherein the distal end of the main body comprises a roof portion (60) covering the main body to form a closed distal end, the roof portion comprising a conically-shaped distal-facing surface that includes a pattern of projections (84) and/or grooves (86) formed thereon.

7. The stopper (38) of claim 6, wherein the conically-shaped distal-facing surface is configured to reduce a dead volume between the distal-facing surface of the stopper and an inner surface of the barrel (12), when the stopper is fully advanced distally within the barrel.

8. The stopper (38) of claim 6 or claim 7, wherein the pattern of projections (84) and/or grooves (86) comprises a star-shaped arrangement of projections extending out from a center point of the roof portion (60), a star-shaped arrangement of grooves extending out from a center point of the roof portion, or a spiraled projection and corresponding spiral groove.

9. The stopper (38) of claim 6 or claim 7, wherein the pattern of projections (84) and/or grooves (86) comprises a plurality of concentric annular projections, with annular grooves formed between each adjacent pair of concentric annular projections; and
wherein, optionally, the pattern of projections (84) and/or grooves (86) further comprises an arrangement of linear grooves extending out from a center point of the roof portion (60) and bisecting the annular grooves, with grooves of the arrangement of linear grooves each spaced apart by 120 degrees.

10. The stopper (38) of claim 8 or claim 9, wherein the pattern of projections (84) and/or grooves (86) comprises the arrangement of linear grooves or the spiraled projection and corresponding spiral groove and the arrangement of linear grooves or the spiral groove is configured to generate a fluid flow that aids in expelling fluid out from the barrel (12) as the stopper is advanced distally within the barrel, and to reduce stickiness between the stopper and the barrel.

11. A stopper (38) for use in an injection device (10) and adapted for attachment with a plunger rod (36) thereof movable within a barrel (12) of the injection device, the stopper comprising:
a main body (40, 52) defining a proximal end and a distal end in a longitudinal direction of the stopper, the proximal end comprising an open proximal end configured to mate with a distal end of the plunger rod, to secure the stopper to the plunger rod; and
a plurality of annular ribs (70, 72, 76) each extending radially outward from an outer surface of the main body and around an outer circumference of the main body, the plurality of annular ribs including at least a first annular rib (70) and a second annular rib (72) spaced from the first annular rib in the longitudinal direction, with an inter-rib region positioned between the first annular rib and the second annular rib;
wherein the proximal end of the main body comprises a proximally-facing shoulder portion (98) that includes a pattern of projections (100) formed thereon and spaced circumferentially about the shoulder portion.

12. The stopper (38) of claim 11, wherein the pattern of projections (100) comprises a plurality of arc-shaped projections formed on the shoulder portion (98) and extending out proximally therefrom, a plurality of wedge-shaped projections formed on the shoulder portion and extending out proximally therefrom, or a continuous circular arrangement of interspersed bumps and depressions formed on the shoulder portion.

13. A stopper (38) for use in an injection device (10) and adapted for attachment with a plunger rod (36) thereof movable within a barrel (12) of the injection device, the stopper comprising:
a main body (40, 52) defining a proximal end and a distal end in a longitudinal direction of the stopper, the proximal end comprising an open proximal end configured to mate with a distal end of the plunger rod, to secure the stopper to the plunger rod; and
at least one annular lip (110) extending radially outward from an outer surface of the main body and around an outer circumference of the main body, so as to interact with the barrel to form a seal therewith,
wherein the at least one annular lip comprises a free outer radial edge configured to deflect responsive to movement of the stopper within the barrel, with the annular lip angled in a proximal direction or a distal direction.

14. The stopper (38) of claim 13, wherein the at least one annular lip (110) is angled in the proximal direction or the distal direction.

15. The stopper (38) of any of claims 13-14, wherein the at least one annular lip (110) comprises a distal lip positioned at the distal end of the main body (40, 52);
wherein, optionally, the at least one annular lip (110) comprises a proximal lip positioned adjacent the proximal end of the main body (40, 52); and
wherein, optionally, the stopper comprises an annular rib (70, 76) extending radially outward from an outer surface of the main body (40, 52) and around an outer circumference of the main body.

16. The stopper (38) of any of claims 13-15, further comprising a plurality of vertical ribs (80) positioned each extending outward from the outer surface of the main body (40, 52), with the plurality of vertical ribs oriented in a longitudinal direction and spaced about the outer circumference of the main body, the plurality of vertical ribs increasing a rigidity of the stopper in the longitudinal direction.
